# EUROPEAN PATENT APPLICATION

(11) **EP 3 125 219 A1**
(43) Date of publication of application: **01.02.2017**
(21) Application number: 16181417.3
(22) Date of filing: 27.07.2016
(51) Int. Cl.: G09B 23/30

(54) **SIMULATED ORGAN**

(30) Priority: 29.07.2015 JP 2015149207
(71) Applicant: Seiko Epson Corporation, Tokyo 160-8801 (JP)
(72) Inventor: Sekino, Hirokazu, Nagano, 392-8502 (JP); Ito, Jiro, Nagano, 392-8502 (JP); Seto, Takeshi, Nagano, 392-8502 (JP)
(74) Representative: Hoffmann Eitle

(57) **Abstract**

A simulated organ includes a simulated parenchyma that simulates a parenchyma cell. The simulated parenchyma has a plurality of colors.

## Description

### BACKGROUND

### 1. Technical Field

The present invention relates to a simulated organ.

### 2. Related Art

In the related art, a structure including a puncture unit and a simulated blood vessel is known as an injection practice device (for example, JP-A-2012-203153). The puncture unit includes a simulated tissue layer corresponding to a simulated parenchyma which simulates a parenchyma (parenchyma cell) of a human body. The simulated blood vessel is arranged so as to penetrate the simulated tissue layer. The simulated tissue layer is configured to include a material to which a skin color pigment is added.

In the related art, the simulated parenchyma (simulated tissue layer) is formed by using a single color such as a skin color. Consequently, in a case where the injection practice device is used for testing of excision performed by using a microscope, light is irregularly reflected due to water contained in the simulated parenchyma, thereby causing a problem in that visibility or a stereoscopic effect is insufficiently achieved in a depth direction when the excision is performed. Without being limited to the testing of the excision performed by using the microscope, this problem is generally common to a test using a simulated organ.

### SUMMARY

An advantage of some aspects of the invention is to improve visibility or a stereoscopic effect so as to improve usability.

The invention can be implemented as the following forms.
(1) An aspect of the invention provides a simulated organ. The simulated organ includes a simulated parenchyma that simulates a parenchyma cell. The simulated parenchyma has a plurality of colors. According to the simulated organ in the aspect, a color difference in the simulated parenchyma can improve visibility or a stereoscopic effect, thereby providing excellent usability.
(2) In the simulated organ according to the aspect, the simulated parenchyma may have different colors in a depth direction. According to this configuration, the visibility or the stereoscopic effect in the depth direction can be improved, thereby enabling the usability to be further improved.
(3) In the simulated organ according to the aspect, a plurality of the colors may be provided with a marble pattern. According to the simulated organ of the aspect with this configuration, the marble pattern can be easily employed by insufficiently mixing a plurality of materials colored in different colors, thereby providing facilitated manufacturing.
(4) In the simulated organ according to the aspect, a plurality of the colors may be provided with a layer of different colors in a depth direction. According to the simulated organ of the aspect with this configuration, the different colors in a layer shape appear in the depth direction. Therefore, the visibility or the stereoscopic effect in the depth direction can be further improved.
(5) In the simulated organ according to the aspect, the simulated organ may further include simulated blood vessel that simulates a blood vessel. A plurality of the colors may be respectively different from a color of the simulated blood vessel. According to the simulated organ of the aspect with this configuration, the simulated organ can include the simulated parenchyma and the simulated blood vessel. Therefore, simulation accuracy can be improved.
(6) In the simulated organ according to the aspect, a configuration may be adopted in which the simulated parenchyma can be excised by a liquid ejected from a liquid ejecting apparatus. According to the simulated organ of the aspect with this configuration, the usability can be improved as the simulated organ used for the liquid ejecting apparatus.

The invention can be implemented in various forms in addition to the above-described configurations. For example, the invention can be implemented as a manufacturing method of the simulated organ.

### BRIEF DESCRIPTION OF THE DRAWINGS

The invention will be described with reference to the accompanying drawings, wherein like numbers reference like elements.
Fig. 1 is a view for schematically describing a configuration of a liquid ejecting apparatus.
Figs. 2A and 2B are views for describing a simulated organ.
Fig. 3 is a process diagram illustrating a manufacturing method of the simulated organ.
Fig. 4 is a view for describing a state in a process of pouring a first material and a second material.
Fig. 5 is a view for describing a state after a hole is opened in the simulated organ by using the liquid ejecting apparatus.
Figs. 6A and 6B are views for describing a simulated organ according to a second embodiment.
Figs. 7A and 7B are views for describing a simulated organ according to a third embodiment.
Fig. 8 is a view for describing a state after a hole is opened in the simulated organ by using a liquid ejecting apparatus in the third embodiment.

### DESCRIPTION OF EXEMPLARY EMBODIMENTS

Hereinafter, embodiments according to the invention will be described. First, a liquid ejecting apparatus used for excising a simulated organ according to the embodiments will be described.

### A. First Embodiment:

### A-1. Configuration of Liquid Ejecting Apparatus:

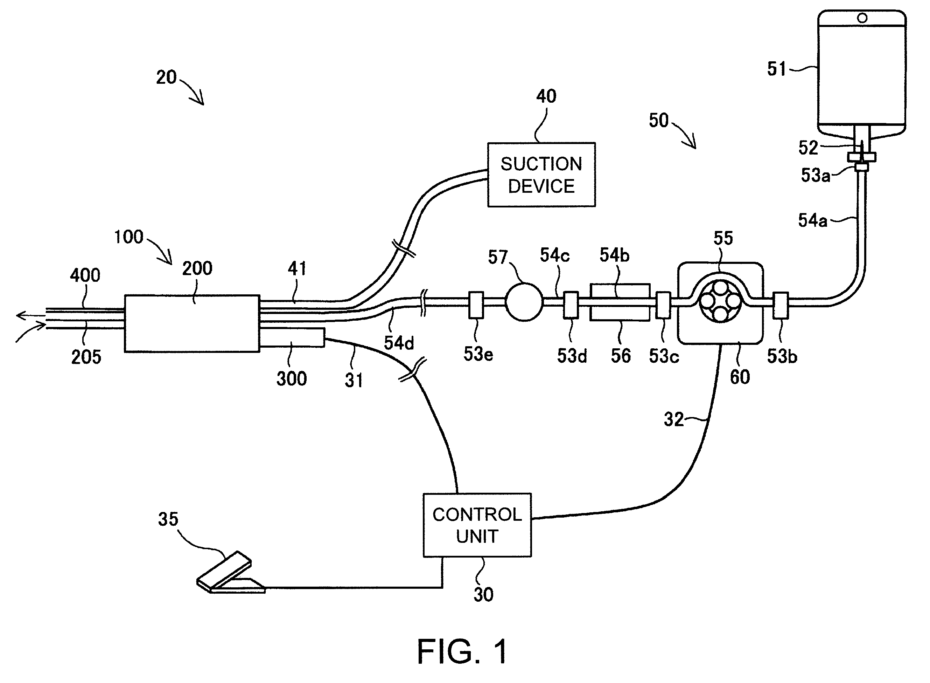

Fig. 1 is a view for schematically describing a configuration of a liquid ejecting apparatus 20. The liquid ejecting apparatus 20 is a medical device used in medical institutions, and has a function to excise a lesion by ejecting a liquid to the lesion.

The liquid ejecting apparatus 20 includes a control unit 30, an actuator cable 31, a pump cable 32, a foot switch 35, a suction device 40, a suction tube 41, a liquid supply device 50, and a handpiece 100.

The liquid supply device 50 includes a water supply bag 51, a spike needle 52, first to fifth connectors 53a to 53e, first to fourth water supply tubes 54a to 54d, a pump tube 55, a clogging detection mechanism 56, and a filter 57. The handpiece 100 includes a nozzle unit 200 and an actuator unit 300. The nozzle unit 200 includes an ejecting tube 205 and a suction pipe 400.

The water supply bag 51 is made of a transparent synthetic resin, and the inside thereof is filled with a liquid (specifically, a physiological saline solution). In the present application, even if a bag is filled with liquids other than the water, the bag is called the water supply bag 51. The spike needle 52 is connected to the first water supply tube 54a via the first connector 53a. If the spike needle 52 is stuck into the water supply bag 51, the liquid filling the water supply bag 51 is in a state where the liquid can be supplied to the first water supply tube 54a.

The first water supply tube 54a is connected to the pump tube 55 via the second connector 53b. The pump tube 55 is connected to the second water supply tube 54b via the third connector 53c. The tube pump 60 pinches the pump tube 55. The tube pump 60 feeds the liquid inside the pump tube 55 to the second water supply tube 54b side from the first water supply tube 54a side.

The clogging detection mechanism 56 detects clogging inside the first to fourth water supply tubes 54a to 54d by measuring pressure inside the second water supply tube 54b.

The second water supply tube 54b is connected to the third water supply tube 54c via the fourth connector 53d. The filter 57 is connected to the third water supply tube 54c. The filter 57 collects foreign substances contained in the liquid.

The third water supply tube 54c is connected to the fourth water supply tube 54d via the fifth connector 53e. The fourth water supply tube 54d is connected to the nozzle unit 200. The liquid supplied through the fourth water supply tube 54d is intermittently ejected from a distal end of the ejecting tube 205 by driving the actuator unit 300. The liquid is intermittently ejected in this way. Accordingly, it is possible to ensure excision capability using a small flow rate.

The ejecting tube 205 and the suction pipe 400 configure a double tube in which the ejecting tube 205 serves as an inner tube and the suction pipe 400 serves as an outer tube. The suction tube 41 is connected to the nozzle unit 200. The suction device 40 performs suction on the inside of the suction pipe 400 through the suction tube 41. The suction is performed on the liquid or excised fragments in the vicinity of the distal end of the suction pipe 400.

The control unit 30 controls the tube pump 60 and the actuator unit 300. Specifically, while the foot switch 35 is stepped on, the control unit 30 transmits a drive signal via the actuator cable 31 and the pump cable 32. The drive signal transmitted via the actuator cable 31 drives a piezoelectric element (not illustrated) included in the actuator unit 300. The drive signal transmitted via the pump cable 32 drives the tube pump 60. Accordingly, while a user steps on the foot switch 35, the liquid is intermittently ejected. While the user does not step on the foot switch 35, the liquid ejection is stopped.

### A-2. Configuration of Simulated Organ:

Next, a simulated organ according to a first embodiment will be described. The simulated organ is also called a phantom, and is an artificial product whose portion is excised by the liquid ejecting apparatus 20 in the present embodiment. The simulated organ according to the embodiment is used in performing a simulated operation for the purpose of a performance evaluation of the liquid ejecting apparatus 20, manipulation practice of the liquid ejecting apparatus 20, and the like.

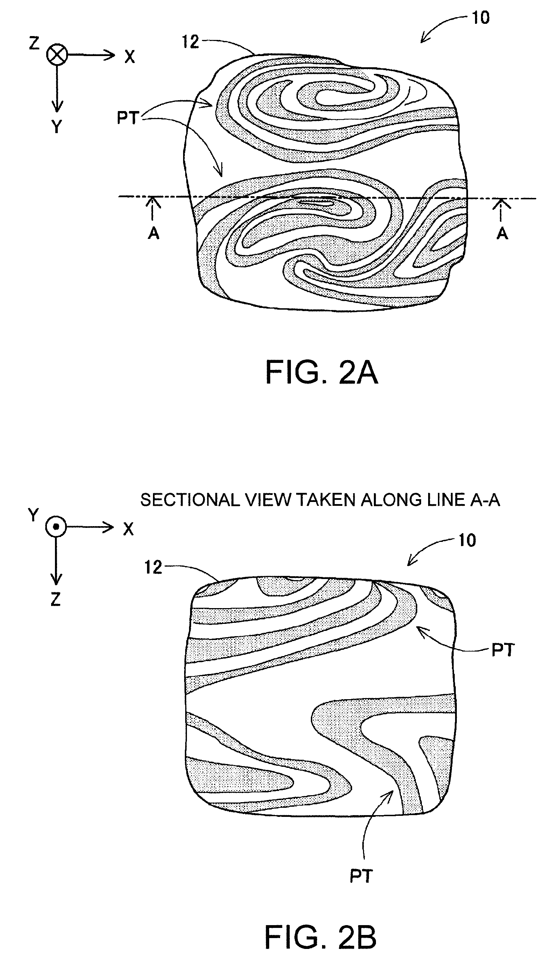

Figs. 2A and 2B are views for describing a simulated organ 10. Fig. 2A illustrates a plan view, and Fig. 2B illustrates a sectional view taken along line A-A in Fig. 2A. In the embodiment, a horizontal plane represents a plane X-Y, and a vertical direction (depth direction) represents a direction Z.

The simulated organ 10 includes a simulated parenchyma 12 and a support member (not illustrated) which supports the simulated parenchyma 12.

The simulated parenchyma 12 is an artificial product that simulates a parenchyma (parenchyma cell) of an organ (for example, a human brain, liver, or the like) of a human body. The parenchyma is a cell which directly relates to a characteristic function of the organ. The simulated parenchyma 12 has an externally block shape which is close to a rectangular shape, and is formed in two colors. For example, the two colors are a white color and an orange color, and are in an insufficiently mixed state. According to the embodiment, the two colors form a marble pattern. In the drawing, a black solid portion is a portion corresponding to the orange color. The marble pattern means a pattern which simulates marble, and appears so that flowing shapes are superimposed on each other or kneaded in a plurality of colors. The simulated parenchyma 12 shows the marble pattern in the horizontal plane direction X-Y as illustrated in Fig. 2A, and also shows the marble pattern in the depth direction Z as illustrated in Fig. 2B.

The above-described two colors are not limited to the white color and the orange color. For example, the two colors can be substituted with a combination of various colors, such as the white color and a skin color, the orange color and the skin color, and the like. In addition, without being necessarily limited to the two colors, the number of colors may be three or more. A plurality of the colors indicating two colors, or three or more colors mean a plurality of different colors. However, in the embodiment, the "different colors" mean that a distance between the two colors (difference degree between the two colors) is separated so as to be visible when the two colors are adjacent to each other.

The simulated parenchyma 12 is supported by the support member (not illustrated). For example, the support member is a metal-made container, and accommodates the simulated parenchyma 12 for support.

### A-3. Manufacturing Method of Simulated Organ:

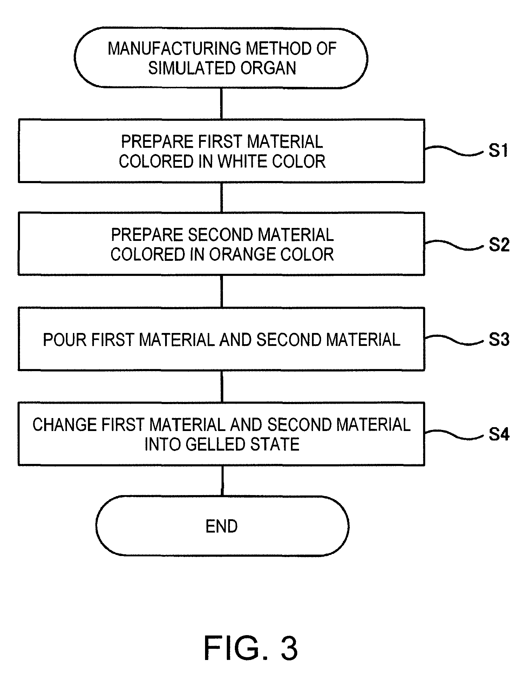

Fig. 3 is a process diagram illustrating a manufacturing method of the simulated organ 10. First, a first material colored in the white color is prepared (Step S1). The embodiment employs polyvinyl alcohol (PVA) as a material of the simulated parenchyma 12. In Step S1, the first material colored with a white colorant (for example, a pigment) mixed with the PVA is prepared.

Next, a second material colored in the orange color is prepared (Step S2). In Step S2, the second material colored with an orange colorant (for example, a pigment) mixed with the PVA is prepared.

Subsequently, the first material prepared in Step S1 and the second material prepared in Step S2 are poured into the container serving as the support member (Step S3).

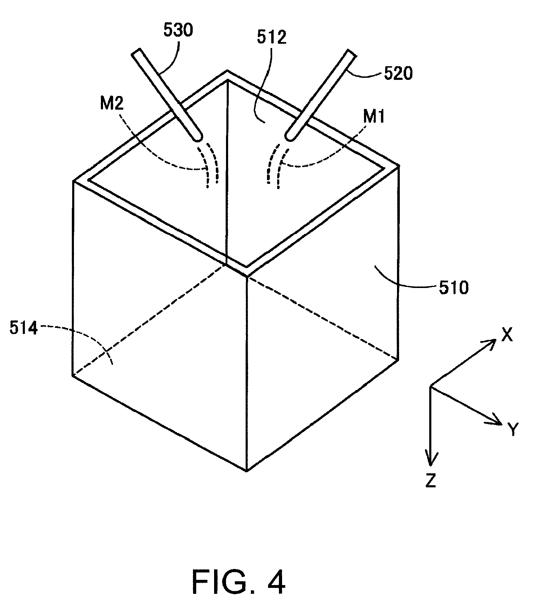

Fig. 4 is a view for describing a state in Step S3. For example, a container 510 has a shape whose upper side has an opening portion 512 and whose lower side has a bottom portion 514. A first injection nozzle 520 and a second injection nozzle 530 are arranged above the opening portion 512. A first material M1 prepared in Step S1 is injected from the first injection nozzle 520 toward the opening portion 512. At the same time, a second material M2 prepared in Step S2 is injected from the second injection nozzle 530 toward the opening portion 512. As a result, the first and second materials M1 and M2 form the above-described marble shape.

After Step S3 in Fig. 3, the container 510 into which the first and second materials M1 and M2 are injected is frozen, thereby changing the first and second materials M1 and M2 so as to be gelled (cured) in a mixed state with a marble pattern (Step S4). In this manner, the simulated parenchyma 12 is formed inside the container 510, and the simulated organ 10 is completely manufactured.

### A-4. Advantageous Effect of Embodiment:

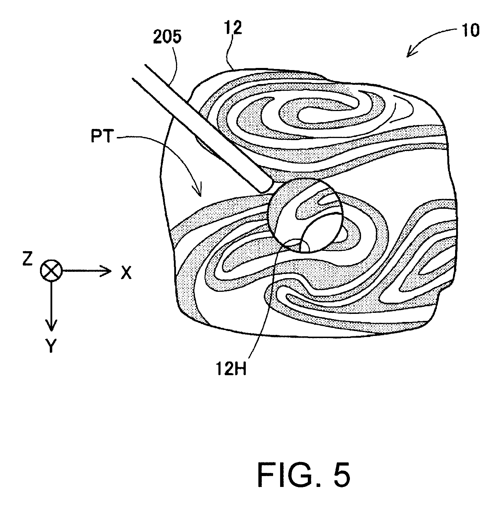

Fig. 5 is a view for describing a state after a hole 12H is opened in the simulated organ 10 by using the liquid ejecting apparatus 20. The simulated organ 10 is illustrated on a plan view. The simulated parenchyma 12 of the simulated organ 10 is gradually excised by a liquid intermittently ejected from the ejecting tube 205 of the liquid ejecting apparatus 20 (Fig. 1), thereby opening the hole 12H in a direction oblique to the depth direction Z in the drawing. In the simulated organ 10 according to the embodiment, the simulated parenchyma 12 has the marble pattern of two colors. Accordingly, as illustrated in the drawing, the marble pattern also appears on a wall surface of the hole 12H. Therefore, in the simulated organ 10, a color difference provided by the marble pattern can improve visibility or a stereoscopic effect in the depth direction, thereby providing excellent usability.

In addition, according to the simulated organ 10 in the embodiment, as illustrated in Fig. 4, the marble pattern can be easily formed by injecting the two materials M1 and M2, thereby providing facilitated manufacturing.

### B. Second Embodiment:

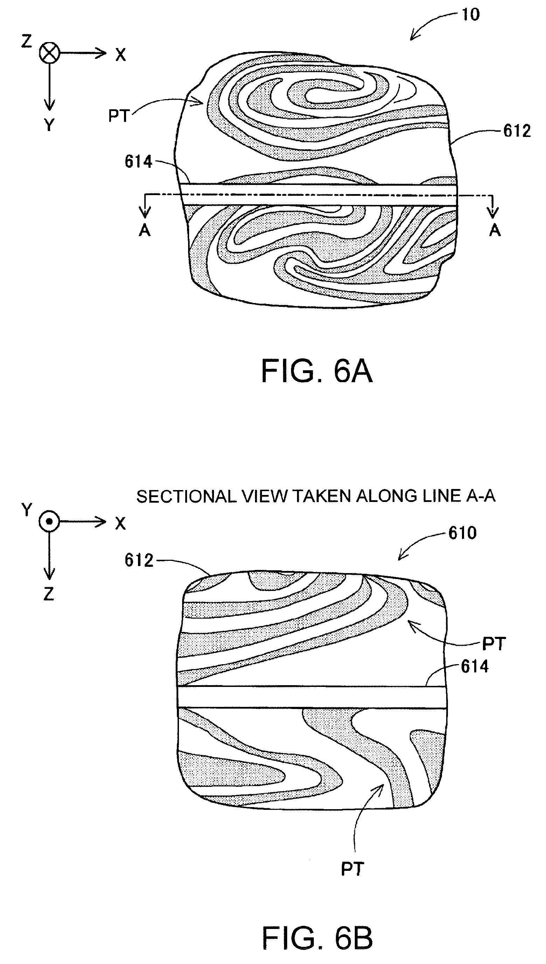

Figs. 6A and 6B are views for describing a simulated organ 610 according to a second embodiment. Fig. 6A illustrates a plan view, and Fig. 6B illustrates a sectional view taken along line A-A in Fig. 6A. That is, Figs. 6A and 6B correspond to Figs. 2A and 2B in the first embodiment.

The simulated organ 610 according to the second embodiment includes a simulated parenchyma 612, a simulated blood vessel 614, and a support member (not illustrated). The simulated parenchyma 612 is the same as the simulated parenchyma 12 included in the simulated organ 10 according to the first embodiment, and has the marble pattern of the white color and the orange color.

The simulated blood vessel 614 is an artificial product that simulates the blood vessel (for example, a human cerebral blood vessel) of a living body, and is formed as a solid member in the embodiment. As a material of the simulated blood vessel 614, PVA is employed. The simulated blood vessel 614 is molded in a red color. The simulated blood vessel 614 is embedded into the simulated parenchyma 612. The simulated blood vessel 614 is a member which has to avoid damage in a simulated operation. The simulated blood vessel 614 can be formed as a hollow member in place of the solid member. The color of the simulated blood vessel 614 may be any color other than the red color, for example, such as a blue color. However, the color of the simulated blood vessel 614 is different from the color used for the simulated parenchyma 612.

The support member (not illustrated) is a metal-made container similar to that of the support member according to the first embodiment, and accommodates the simulated parenchyma 612 having the simulated blood vessel 614 embedded therein, thereby supporting the simulated parenchyma 612.

Similarly to the simulated organ 10 according to the first embodiment, in the simulated organ 610 according to the second embodiment configured as described above, a color difference in the simulated parenchyma 612 can improve visibility or a stereoscopic effect in the depth direction, thereby providing excellent usability. In addition, in the simulated organ 610, each color (white and orange) in the simulated parenchyma 612 is different from the color (red) of the simulated blood vessel 614. Accordingly, the visibility of the simulated blood vessel 614 is not impaired. In addition, the simulated organ 610 can be configured to include the simulated parenchyma 612 and the simulated blood vessel 614. Therefore, simulation accuracy can be improved.

According to the second embodiment, the simulated parenchyma 612 employs the white color and the orange color. However, as long as a color different from the color of the simulated blood vessel 614 is used, a configuration using any color may be adopted. In addition, without being limited to two, the number of colors to be used may be three or more. In addition, according to the second embodiment, one simulated blood vessel 614 is included in the simulated organ 610. However, alternatively, two or more simulated blood vessels 614 may be included therein.

### C. Third Embodiment:

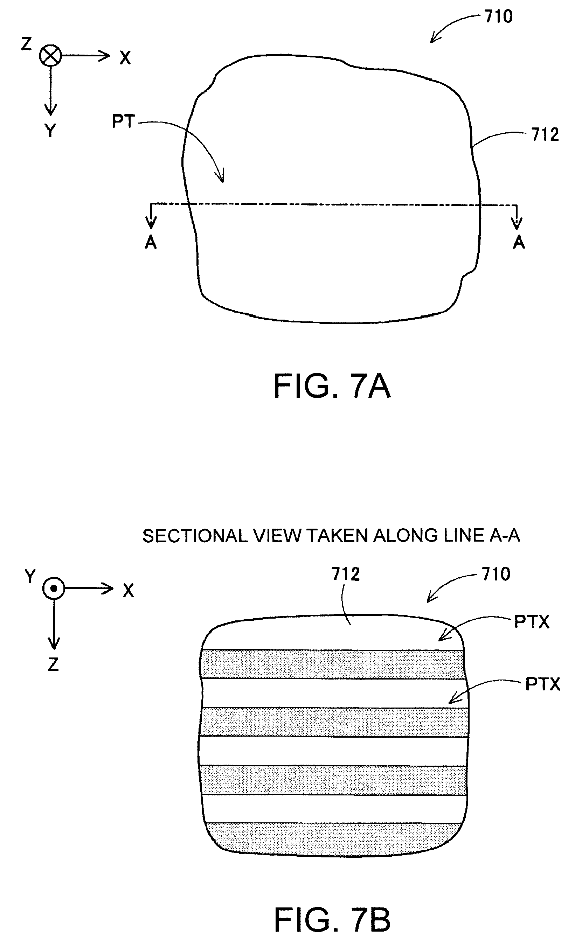

Figs. 7A and 7B are views for describing a simulated organ 710 according to a third embodiment. Fig. 7A illustrates a plan view, and Fig. 7B illustrates a sectional view taken along line A-A in Fig. 7A. That is, Figs. 7A and 7B correspond to Figs. 2A and 2B in the first embodiment.

The first embodiment and the second embodiment adopt a configuration in which a plurality of colors in the simulated parenchyma 12 or 612 are provided with the marble pattern. In contrast, according to the third embodiment, a simulated parenchyma 712 included in the simulated organ 710 is formed in a single color in the plane direction X-Y as illustrated in Fig. 7A, but is formed in a layer shape of different colors in the depth direction Z as illustrated in Fig. 7B. For example, as the different colors, the white color and the orange color are used. In the drawing, a black solid portion is a portion corresponding to the orange color. Without being limited to the two colors, the different colors may be three or more colors. Without being limited to the combination of the white color and the orange color, the combination of the colors can be substituted with a combination of various colors, such as the white color and the skin color, the orange color and the skin color, and the like.

Similarly to the manufacturing method (Fig. 3) of the simulated organ according to the first embodiment, according to a manufacturing method of the simulated organ 710, a first material colored in the white color and a second material colored in the orange color are prepared. Next, respectively determined amounts of the first material and the second material are alternately injected and stacked on each other, thereby forming a layer-shaped pattern.

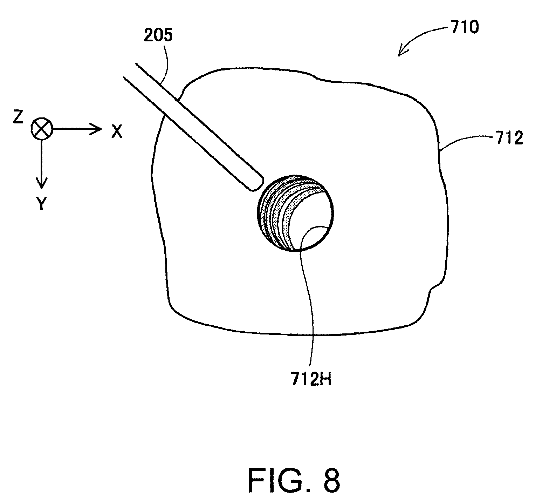

Fig. 8 is a view for describing a state after a hole 712H is opened in the simulated organ 710 by using the liquid ejecting apparatus 20. The simulated organ 710 is illustrated on a plan view. The simulated parenchyma 712 of the simulated organ 710 is gradually excised by a liquid intermittently ejected from the ejecting tube 205 of the liquid ejecting apparatus 20 (Fig. 1), thereby opening the hole 712H in a direction oblique to the depth direction Z in the drawing. In the simulated organ 710 according to the embodiment, the simulated parenchyma 712 is formed in the layer shape of different colors in the depth direction Z. Accordingly, as illustrated in the drawing, the layer-shaped pattern also appears on a wall surface of the hole 712H. Therefore, similarly to the first embodiment, in the simulated organ 710 according to the third embodiment, a color difference provided by the layer-shaped pattern can improve visibility or a stereoscopic effect in the depth direction, thereby providing excellent usability.

As a modification example of the third embodiment, a configuration may be adopted in which the simulated organ 710 is formed in a single color in the depth direction Z and is formed in the layer shape of different colors in the plane direction X-Y.

### D. Modification Example:

Without being limited to the respective embodiments and modification examples thereof, the invention can be embodied in various forms within the scope not departing from the gist. For example, the invention can be modified as follows.

### Modification Example 1:

A primary material of the simulated parenchyma included in the simulated organ is the PVA, but is not limited thereto. For example, urethane or a rubber-based material other than urethane may also be used.

### Modification Example 2:

The material of the simulated blood vessel included in the simulated organ according to the second embodiment is the PVA, but is not limited thereto. For example, a synthetic resin other than the PVA (for example, urethane) may also be used, or a natural resin may also be used.

### Modification Example 3:

The simulated parenchyma may be manufactured by using injection deposition (3D printing using an ink jet method). In addition, the simulated blood vessel may be manufactured by using 3D printing. Furthermore, the simulated parenchyma and the simulated blood vessel may be collectively manufactured by using 3D printing.

### Modification Example 4:

A shape of the simulated parenchyma is configured to be a shape close to a rectangular shape, but is not limited thereto. For example, other shapes such as a cylindrical shape, a conical shape, a truncated cone shape, and the like may be used.

### Modification Example 5:

The simulated organ may be excised by using those which other than the liquid intermittently ejected from the liquid ejecting apparatus. For example, the simulated organ may be excised by using a continuously ejected liquid, or may be excised by using a liquid provided with excision capability using an ultrasound or an optical maser. Alternatively, the simulated organ may be excised by using a metal scalpel.

### Modification Example 6:

The embodiments adopt a configuration in which the piezoelectric element is used as the actuator. However, the embodiments may adopt a configuration in which the liquid is ejected by using the optical maser, a configuration in which the liquid is ejected by a heater generating air bubbles in the liquid, or a configuration in which the liquid is ejected by a pump pressurizing the liquid. According to the configuration in which the liquid is ejected by using the optical maser, the optical maser emits radiation to the liquid so as to generate the air bubbles in the liquid, and increased pressure of the liquid which is caused by generating the air bubbles is utilized.

Without being limited to the embodiments, the application examples, and the modification examples which are described above, the invention can be implemented according to various configurations within the scope not departing from the gist of the invention. For example, technical features in the embodiments, the application examples, and the modification examples which correspond to technical features according to each aspect described in the summary of the invention can be appropriately replaced or combined with each other in order to partially or entirely solve the previously described problem or in order to partially or entirely achieve the previously described advantageous effects. If any one of the technical features is not described herein as essential, the technical feature can be appropriately omitted.

## Claims

1. A simulated organ comprising:
a simulated parenchyma that simulates a parenchyma cell,
wherein the simulated parenchyma has a plurality of colors.

2. The simulated organ according to claim 1, wherein the simulated parenchyma has different colors in a depth direction.

3. The simulated organ according to claim 1 or claim 2,
wherein the plurality of the colors are provided with a marble pattern.

4. The simulated organ according to claim 1 or claim 2,
wherein the plurality of the colors are provided with a layer of different colors in a depth direction.

5. The simulated organ according to any of claims 1 to 4, further comprising:
a simulated blood vessel that simulates a blood vessel,
wherein the plurality of the colors are respectively different from a color of the simulated blood vessel.

6. The simulated organ according to any of claims 1 to 5,
wherein the simulated parenchyma can be excised by a liquid ejected from a liquid ejecting apparatus.

7. The simulated organ according to any of claims 1 to 6, wherein the simulated parenchyma has an externally block shape which is close to a rectangular shape, and if formed in two colors.

8. The simulated organ according to claim 7, wherein the two colors are a white color and an orange color, and are in an insufficiently mixed state.

9. The simulated organ according to any of claims 3 to 8, wherein the marble pattern is a pattern which simulates marble, and appears so that flowing shapes are superimposed on each other or kneaded in a a plurality of colors.

10. The simulated organ according to any of claims 7 to 9, wherein the two colors comprise a combination of various colors, such as a white color and skin color, an orange color and a skin color, and the like.

11. The simulated organ according to any of claims 2 to 10, wherein the different colors means that a distance between the two colors (difference degree between the two colors) is separated so as to be visible when the two colors are adjacent to each other.

12. The simulated organ according to any of claims 1 to 10, wherein the simulated organ comprises a first material colored in a white color and second material colored in an orange color, where respectively determined amounts of the first material and the second material are alternately inj ected an stacked on each other, thereby forming a layer-shaped pattern.

13. The simulated organ according to any of claims 1 to 12, wherein a primary material of the simulated parenchyma included in the simulated organ is PVA, urethane or a rubber-based material.

14. The simulated organ according to any of claims 1 to 13, wherein the material of the simulated blood vessel is PVA, a synthetic resin other than PVA, for example, urethane, or a natural resin.

15. The simulated organ according to any of claims 1 to 14, wherein the simulated parenchyma is configured to be a shaped close to a rectangular shape, or other shapes such as a cylindrical shape, a conical shape, a truncated cone shape, and the like.
